(19) Europäisches Patentamt
European Patent Office
Office européen des brevets

(11) EP 4 736 978 A2

(12) DEMANDE DE BREVET EUROPEEN

(43) Date de publication:
**06.05.2026 Bulletin 2026/19**

(21) Numéro de dépôt: **25203190.1**

(22) Date de dépôt: **18.09.2025**

(51) Classification Internationale des Brevets (IPC):
***B01D 11/02*** $^{(2006.01)}$ ***B01D 11/04*** $^{(2006.01)}$

(52) Classification Coopérative des Brevets (CPC):
**B01D 11/0288; B01D 11/0492**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **19.09.2024 FR 2409998**

(71) Demandeurs:
- **Commissariat à l’Energie Atomique et aux Energies Alternatives**
  **75015 Paris (FR)**
- **Centre National de la Recherche Scientifique**
  **75016 Paris (FR)**
- **université Clermont Auvergne**
  **63000 Clermont Ferrand (FR)**

(72) Inventeurs:
- **VERGNORY, Pauline**
  **91191 GIF-SUR-YVETTE CEDEX (FR)**
- **BERTRAND, Guillaume**
  **91191 GIF-SUR-YVETTE CEDEX (FR)**
- **ANDANSON, Jean-Michel**
  **63000 CLERMONT-FERRAND (FR)**

(74) Mandataire: **Gevers & Orès**
**Immeuble le Palatin 2**
**3 Cours du Triangle**
**CS 80165**
**92939 Paris La Défense Cedex (FR)**

# (54) MELANGES EUTECTIQUES AROMATIQUES ET LEURS UTILISATIONS EN TANT QUE SOLVANTS, NOTAMMENT EN SEPARATION LIQUIDE LIQUIDE

(57) La présente invention concerne des mélanges eutectiques aromatiques et polyaromatiques et leurs utilisations en tant que solvants, notamment en séparation liquide-liquide.



Processed by Luminess, 75001 PARIS (FR)

## Description

La présente invention concerne des mélanges eutectiques aromatiques et polyaromatiques et leurs utilisations en tant que solvants, notamment en séparation liquide-liquide.

L'extraction à l'aide d'un solvant organique est une méthode couramment utilisée, notamment en génie chimique et en chimie analytique, pour extraire, mais également séparer et purifier, un ou des composés chimiques. Il s'agit en général de prélever une ou plusieurs espèces chimiques (ou extractibles) d'un mélange liquide, solide ou d'une suspension d'un solide dans un liquide.

Typiquement, un moyen d'extraction est utilisé pour extraire sélectivement un ou plusieurs composés d'un mélange initial, sur la base de propriétés chimiques ou physiques. Le moyen d'extraction n'est pas ou peu miscible avec les composants principaux du mélange initial, et le composé à extraire possède plus d'affinité avec le moyen d'extraction qu'avec les composants principaux du mélange initial.

L'opération d'extraction se déroule en deux parties : une première partie de transfert du composé à extraire entre le mélange initial et le moyen d'extraction ; une deuxième partie de séparation du moyen d'extraction du mélange principal.

Ce moyen d'extraction est généralement un solvant organique.

Dans une extraction liquide-liquide, une (ou plusieurs) substance dissoute dans un solvant (phase d'alimentation, généralement aqueuse), est extraite à l'aide d'un autre solvant, appelé phase solvant d'extraction, généralement organique) dans lequel elle est plus soluble. Le solvant initial et le solvant d'extraction ne doivent pas être miscibles. Cette technique repose sur la solubilité différentielle des substances à extraire dans deux phases liquides non miscibles.

Dans une extraction solide-liquide, il s'agit d'extraire une substance présente dans un solide pour la faire passer dans un solvant liquide. La macération, l'infusion et la technique de décoction sont des méthodes d'extraction solide-liquide.

Toutefois, les solvants organiques traditionnellement utilisés dans ce contexte sont généralement hautement inflammables ce qui complique leur manipulation et leur stockage et les rendent risqués. En effet, ces solvants organiques ont typiquement des points d'éclair (« flash point ») assez faibles, typiquement <100°C. Ceci représente un risque majeur pour la sécurité de ces applications, qui sont susceptibles d'utiliser un grand volume de ces solvants.

En outre, les solvants organiques sont souvent volatils et contribuent à la pollution de l'air. Leur mauvaise gestion peut entraîner la contamination des sols et des eaux. Ils menacent également la santé des opérateurs concernés, incluant des maux de tête, des nausées, et dans des cas graves, des effets neurologiques et/ou cancérogènes/mutagènes.

La recherche de solvants plus sûrs, moins toxiques et plus respectueux de l'environnement pour minimiser les risques pour la santé et l'environnement tout en maintenant voire améliorant l'efficacité des procédés d'extraction est une priorité. Pour pallier ce problème de volatilité, les chercheurs ont créé de nombreux solvants eutectique polaire et apolaire. Ces solvants eutectiques sont des mélanges de molécules avec des points de liquéfactions proches de la température ambiante. Cependant, ces solvants eutectiques n'ont que peu d'affinité pour bon nombre de molécules, notamment pour les molécules comprenant des résidus aromatiques.

L'invention a ainsi pour but de fournir des compositions permettant de lever, en tant que solvant, les inconvénients précités et notamment de fournir pour la première fois un solvant eutectique aromatique liquide à température ambiante.

Un objectif de l'invention est de fournir des compositions peu inflammables, ayant en particulier des points d'éclair > 100 °C, diminuant les risques précités, tout en pouvant être utilisées en tant que solvant aromatique.

Un autre objectif de l'invention est de fournir de telles compositions, lesquelles sont au moins équivalentes, voire plus efficaces que les solvants traditionnellement utilisés, par exemple le toluène, notamment en extraction liquide/liquide.

## OBJET DE L'INVENTION

Ainsi, selon un premier aspect, l'invention concerne une composition constituée de ou comprenant au moins deux composés choisis parmi :

- Les di-tert-butyl-benzènes;
- Les biphényles et terphényles, optionnellement substitués par deux groupes choisis indépendamment parmi les alkyles linéaires ou branchés en $C_1$ à $C_6$, les terphényles étant notamment des ortho-terphényles, méta-terphényles, ou para-terphényles, en particulier des para-terphényles ou des ortho-terphényles ;
- Les hydrocarbures aromatiques polycycliques choisis parmi naphtalènes, acénaphtènes, anthracènes, pyrènes, lesquels sont optionnellement substitués par deux groupes choisis indépendamment parmi les alkyles linéaires ou branchés en $C_1$ à $C_6$ ;
- Les fluorènes et carbazoles, lesquels sont optionnellement substitués par deux ou quatre groupes choisis indépendamment parmi les alkyles linéaires ou branchés en $C_1$ à $C_6$ ;
- Les 2,5-diphenyloxazoles, optionnellement substitués par deux groupes choisis indépendamment parmi les alkyles

linéaires ou branchés en $C_1$ à $C_6$ ;

- Les composés de formule (I) :

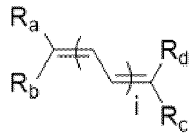

,

dans laquelle :

i est égal à 0 ou 1 ;
$R_a$, $R_b$, $R_c$ et $R_d$ sont indépendamment choisis parmi H et phényle, au moins deux des groupes $R_a$, $R_b$, $R_c$ et $R_d$ étant un phényle ;
lesdits composés de formule (I) étant optionnellement substitués, en particulier sur les groupes phényle, par deux groupes choisis indépendamment parmi les alkyles linéaires ou branchés en $C_1$ à $C_6$ ;

- Le triphénylméthane, optionnellement substitué, en particulier sur chaque groupe phényle, par un groupe choisi indépendamment parmi les alkyles linéaires ou branchés en $C_1$ à $C_6$ ;
- La tribenzylamine, optionnellement substituée, en particulier sur chaque groupe phényle, par un groupe choisi indépendamment parmi les alkyles linéaires ou branchés en $C_1$ à $C_6$ ;
- Le 2,5-dibenzylphénol, optionnellement substitué, en particulier sur chaque atome de carbone en alpha des groupes phényle, par un ou deux groupes choisis indépendamment parmi les alkyles linéaires ou branchés en $C_1$ à $C_6$, le 2,5-dibenzylphénol substitué étant en particulier le 2,5-bis(2-phénylpropan-2-yl)phénol ;

ladite composition n'étant pas constituée du couple biphényle et naphtalène.

De façon surprenante, il a été mis en évidence par les Inventeurs que ces composés ont un point de fusion >60°C individuellement, mais qui une fois au sein d'une composition selon l'invention, ont un point de fusion plus bas, voire liquide à température ambiante, au sein d'un eutectique purement aromatique, voire poly-aromatiques.

Et des composés s'avèrent être d'excellents solvants, notamment pour des molécules comprenant des résidus aromatiques. Par exemple, les composés de l'invention sont plus efficaces que le toluène dans la séparation liquide-liquide impliquant ces molécules.

Selon un mode de réalisation, la composition selon l'invention est eutectique.

Selon un mode de réalisation, les au moins deux composés sont notamment choisis parmi les composés suivants :

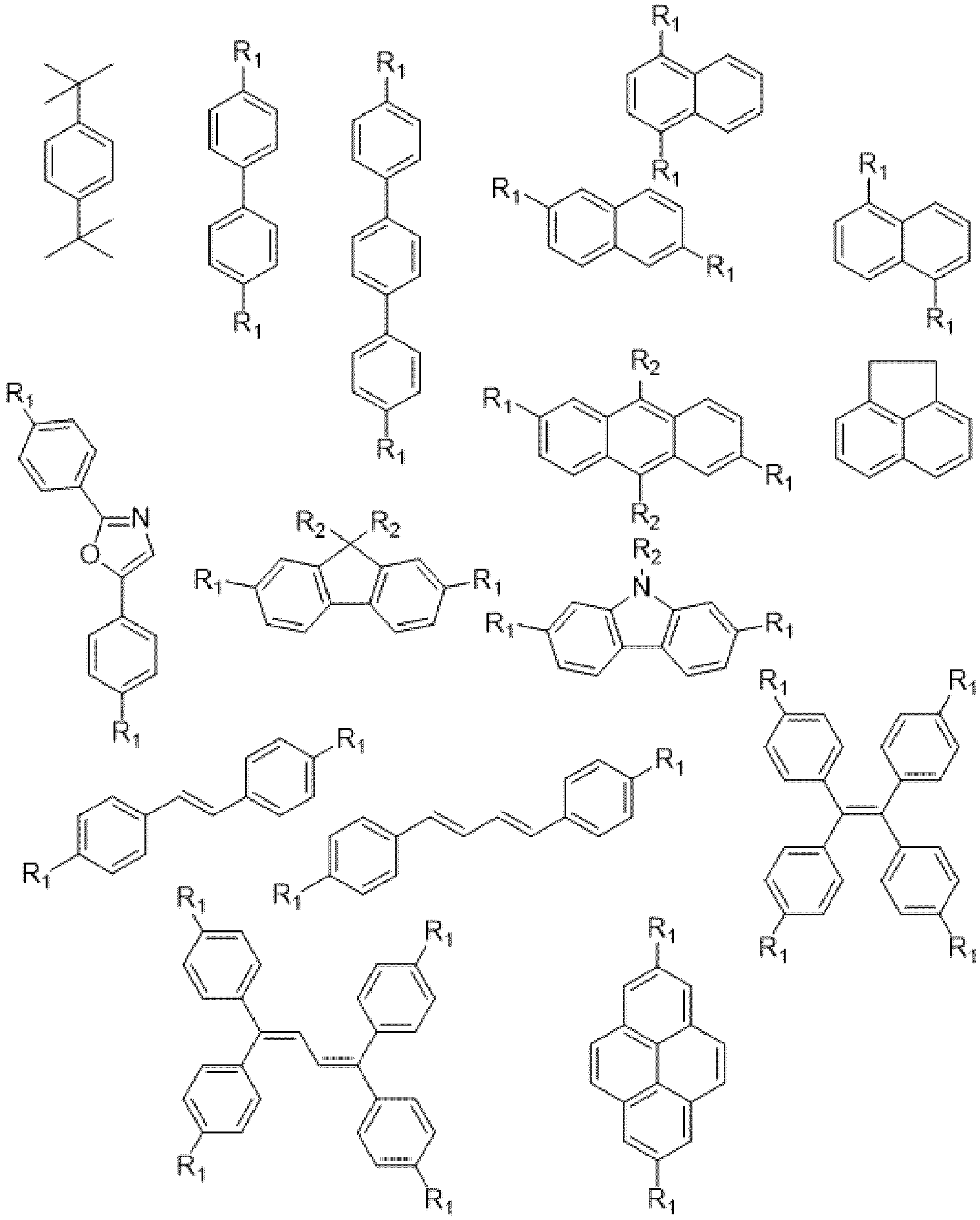

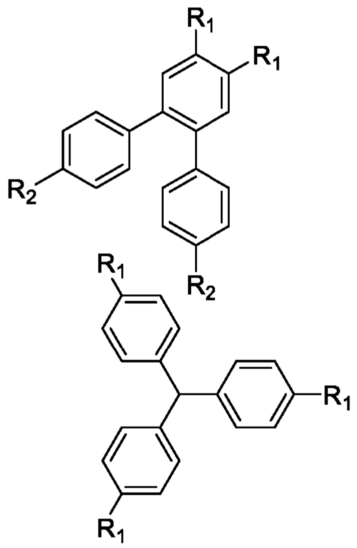

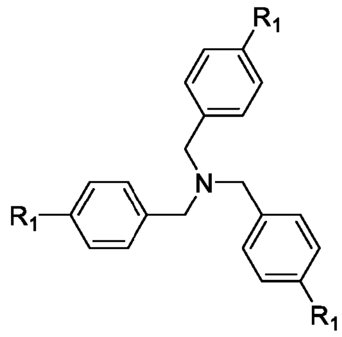

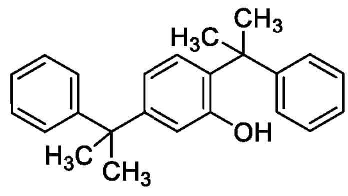

dans lesquelles $R_1$ et $R_2$ sont choisis indépendamment d'un composé à l'autre,
$R_1$ étant choisi parmi H et les alkyles linéaires ou branchés en $C_1$ à $C_6$, et $R_2$ étant choisi parmi H et les alkyles linéaires ou branchés en $C_1$ à $C_6$.

Selon un mode de réalisation, l'invention concerne une composition constituée de ou comprenant au moins deux composés choisis parmi :

- Les di-tert-butyl-benzènes;
- Les biphényles et terphényles, optionnellement substitués par deux groupes choisis indépendamment parmi les alkyles linéaires ou branchés en $C_1$ à $C_6$, les terphényles étant notamment des ortho-terphényles, méta-terphényles, ou para-terphényles, en particulier des para-terphényles ou des ortho-terphényles ;
- Les hydrocarbures aromatiques polycycliques choisis parmi naphtalènes, acénaphtènes, anthracènes, lesquels sont optionnellement substitués par deux groupes choisis indépendamment parmi les alkyles linéaires ou branchés en $C_1$ à $C_6$ ;
- Les fluorènes et carbazoles, lesquels sont optionnellement substitués par deux ou quatre groupes choisis indépendamment parmi les alkyles linéaires ou branchés en $C_1$ à $C_6$ ;
- Les 2,5-diphenyloxazoles, optionnellement substitués par deux groupes choisis indépendamment parmi les alkyles linéaires ou branchés en $C_1$ à $C_6$ ;
- Le triphénylméthane, optionnellement substitué, en particulier sur chaque groupe phényle, par un groupe choisi indépendamment parmi les alkyles linéaires ou branchés en $C_1$ à $C_6$ ;
- La tribenzylamine, optionnellement substituée, en particulier sur chaque groupe phényle, par un groupe choisi indépendamment parmi les alkyles linéaires ou branchés en $C_1$ à $C_6$ ;
- Le 2,5-dibenzylphénol, optionnellement substitué, en particulier sur chaque atome de carbone en alpha des groupes phényle, par un ou deux groupes choisis indépendamment parmi les alkyles linéaires ou branchés en $C_1$ à $C_6$, le 2,5-dibenzylphénol substitué étant en particulier le 2,5-bis(2-phénylpropan-2-yl)phénol ;

ladite composition n'étant pas constituée du couple biphényle et naphtalène.

Selon un mode de réalisation, les au moins deux composés sont notamment choisis parmi les composés suivants :

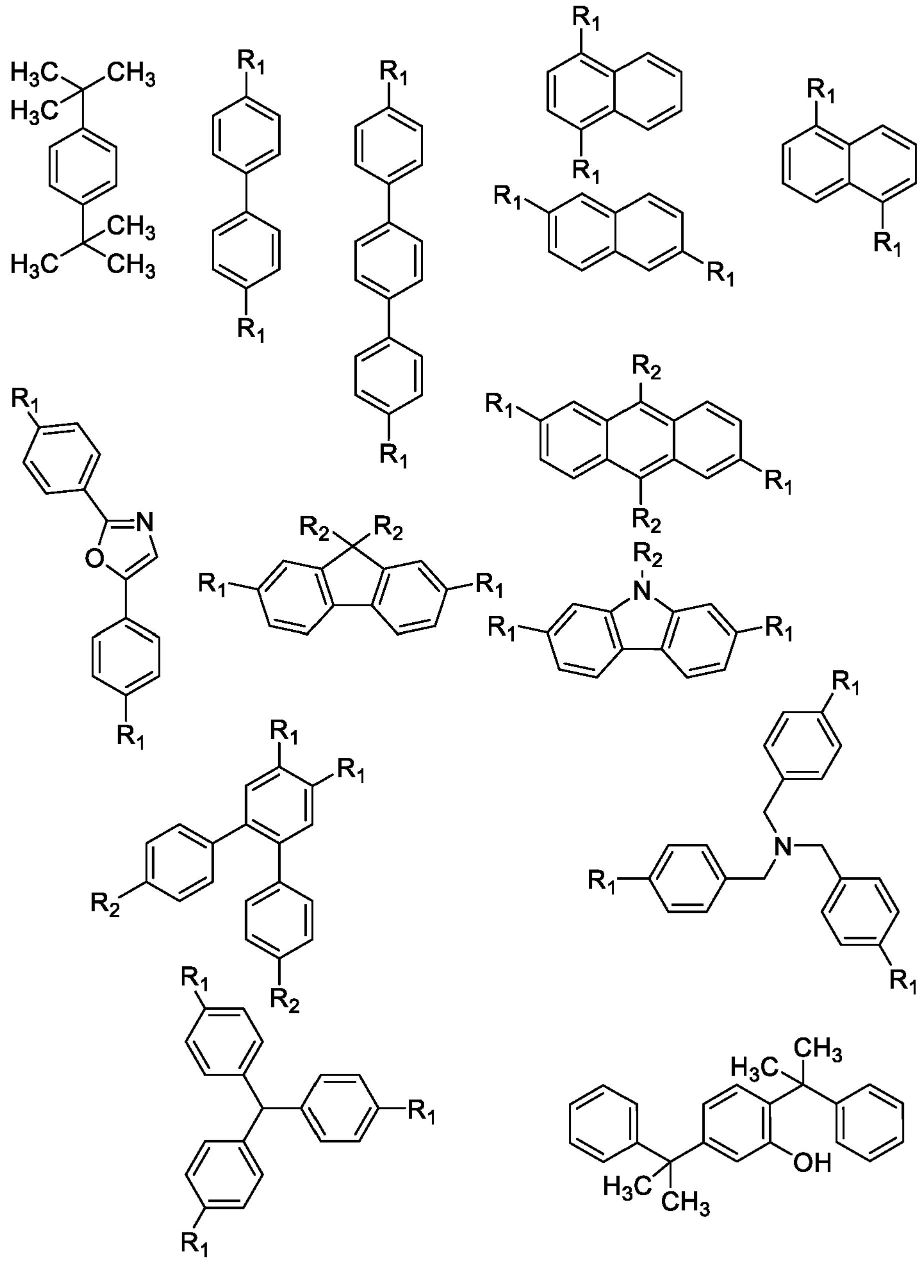

Selon un mode de réalisation, la composition de l'invention est binaire, tertiaire, quaternaire, quinquénaire et ainsi de suite selon le nombre de composants.

Selon un mode de réalisation, la composition de l'invention est binaire, tertiaire, quaternaire, quinquénaire.

Selon un mode de réalisation, la composition de l'invention est :

- binaire, et les deux composés de ladite composition sont présents dans celle-ci à hauteur de 20 à 80 %molaire, en particulier à hauteur de 40 à 60 %molaire ;
- tertiaire, et les trois composés de ladite composition sont présents dans celle-ci à hauteur de 10 à 55 %molaire, en particulier à hauteur de 25 à 40 %molaire ;
- quaternaire, et les quatre composés de ladite composition sont présents dans celle-ci à hauteur de 5 à 45 %molaire, en particulier à hauteur de 22 à 40 %molaire pour trois d'entre eux, et à hauteur de 6 à 34 %molaire pour le dernier ; ou
- quinquénaire, et les cinq composés de ladite composition sont présents dans celle-ci à hauteur de 5 à 45 %molaire.

Selon un autre aspect, l'invention concerne également l'utilisation d'une composition en tant que solvant, ladite composition étant constituée de ou comprenant au moins deux composés choisis parmi :

- Les di-tert-butyl-benzènes;

- Les biphényles et terphényles, optionnellement substitués par deux groupes choisis indépendamment parmi les alkyles linéaires ou branchés en $C_1$ à $C_6$, les terphényles étant notamment des ortho-terphényles, méta-terphényles, ou para-terphényles, en particulier des para-terphényles ou des ortho-terphényles ;
- Les hydrocarbures aromatiques polycycliques choisis parmi naphtalènes, acénaphtènes, anthracènes, pyrènes, lesquels sont optionnellement substitués par deux groupes choisis indépendamment parmi les alkyles linéaires ou branchés en $C_1$ à $C_6$ ;
- Les fluorènes et carbazoles, lesquels sont optionnellement substitués par au moins deux groupes choisis indépendamment parmi les alkyles linéaires ou branchés en $C_1$ à $C_6$ ;
- Les 2,5-diphenyloxazoles, optionnellement substitués par deux groupes choisis indépendamment parmi les alkyles linéaires ou branchés en $C_1$ à $C_6$ ;
- Les composés de formule (I) :

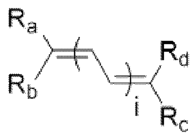

,

dans laquelle :

i est égal à 0 ou 1 ;
$R_a$, $R_b$, $R_c$ et $R_d$ sont indépendamment choisis parmi H et phényle, au moins deux des groupes $R_a$, $R_b$, $R_c$ et $R_d$ étant un phényle ;
lesdits composés de formule (I) étant optionnellement substitués, en particulier sur les groupes phényle, par deux groupes choisis indépendamment parmi les alkyles linéaires ou branchés en $C_1$ à $C_6$ ;

- Le triphénylméthane, optionnellement substitué, en particulier sur chaque groupe phényle, par un groupe choisi indépendamment parmi les alkyles linéaires ou branchés en $C_1$ à $C_6$ ;
- La tribenzylamine, optionnellement substituée, en particulier sur chaque groupe phényle, par un groupe choisi indépendamment parmi les alkyles linéaires ou branchés en $C_1$ à $C_6$ ;
- Le 2,5-dibenzylphénol, optionnellement substitué, en particulier sur chaque atome de carbone en alpha des groupes phényle, par un ou deux groupes choisis indépendamment parmi les alkyles linéaires ou branchés en $C_1$ à $C_6$, le 2,5-dibenzylphénol substitué étant en particulier le 2,5-bis(2-phénylpropan-2-yl)phénol.

De façon surprenante, il a été mis en évidence par les Inventeurs que ces composés ont un point de fusion >60°C individuellement, mais qui une fois au sein d'une composition selon l'invention, ont un point de fusion plus bas, voire liquide à température ambiante, au sein d'un eutectique purement aromatique, voire poly-aromatiques.

Ces compositions eutectiques purement aromatique, voire poly-aromatiques s'avèrent être d'excellents solvants, aux caractéristiques physico-chimiques uniques, notamment pour des molécules comprenant des résidus aromatiques. Par exemple, les composés de l'invention sont plus efficaces que le toluène dans la séparation liquide-liquide impliquant ces molécules.

Selon un mode de réalisation, ladite composition est dénuée d'un autre solvant, l'autre solvant étant différent de tous les composés définis précédemment et de leurs mélanges.

Selon un mode de réalisation, ladite utilisation de l'invention est une utilisation de la composition telle que définie précédemment pour la séparation liquide-liquide, la séparation liquide-liquide-liquide, la séparation solide liquide, ou la séparation solide-liquide-liquide.

Selon un mode de réalisation, ladite utilisation de l'invention est une utilisation de la composition telle que définie précédemment pour la séparation liquide-liquide, l'une des phases liquides étant une phase aqueuse, l'autre phase liquide comprenant ou étant constituée de ladite composition.

Selon un mode de réalisation, ladite utilisation de l'invention est une utilisation de la composition telle que définie précédemment pour l'extraction d'un composé, ledit composé étant en particulier choisi parmi les composés organiques et les métaux, les composés organiques étant notamment des composés aromatiques ou hétéroaromatiques, ou comprenant des groupes aromatiques ou hétéroaromatiques,

Selon un mode de réalisation, lesdits composés organiques, notamment aromatiques ou hétéroaromatiques, ou comprenant des groupes aromatiques ou hétéroaromatiques sont choisis parmi :

- Les composés phytosanitaires,

- Les polluants organiques, en particulier persistants, par exemple les dioxines,
- Les microplastiques,
- Les médicaments,
- Les colorants, et
- Le noir de carbone.

## DESCRIPTION DETAILLEE DE L'INVENTION

### Définitions

Tel qu'on l'entend ici, les plages de valeur sous forme de « x-y » ou « de x à y » ou « entre x et y » incluent les bornes x et y ainsi que les entiers compris entre ces bornes, et les nombres réels positifs compris entre ces bornes et/ou entiers. A titre d'exemple, « 1-5 », ou « de 1 à 5 » ou « entre 1 et 5 » désignent les entiers 1, 2, 3, 4 et 5. Les modes de réalisations préférés incluent chaque entier pris individuellement dans la plage de valeur, ainsi que toute sous-combinaison de ces entiers. A titre d'exemple, les valeurs préférées pour « 1-5 » peuvent comprendre les entiers 1, 2, 3, 4, 5, 1-2, 1-3, 1-4, 1-5, 2-3, 2-4, 2-5, etc.

Tel qu'il est utilisé ici, le terme "alkyle" désigne un groupe alkyle à chaîne linéaire ou ramifiée, ayant le nombre d'atomes de carbone indiqué avant ledit terme, notamment 1 à 4 atomes de carbone, tel que méthyle, éthyle, propyle, isopropyle, butyle, isobutyle, sec-butyle, tert-butyle, etc. Ainsi, une expression telle que "alkyle en en $C_1$ à $C_4$" désigne un radical alkyle contenant de 1 à 4 atomes de carbone.

Par « ladite composition n'est pas constituée de biphényle et naphtalène », on entend notamment que ladite composition n'est pas un mélange constitué de biphényle non substitué et de naphtalène non substitué. En particulier, ladite composition n'étant pas constituée de biphényle, optionnellement substitué par deux groupes choisis indépendamment parmi les alkyles linéaires ou branchés en $C_1$ à $C_6$, et de naphtalène, optionnellement substitué par deux groupes choisis indépendamment parmi les alkyles linéaires ou branchés en $C_1$ à $C_6$.

Les compositions de l'invention sont des eutectiques.

Par « eutectique », on entend notamment un mélange de deux ou plusieurs substances qui a une température de fusion plus basse que celle de chacune des substances individuelles.

L'invention concerne notamment une composition constituée de ou comprenant au moins deux composés choisis parmi :

- Les biphényles et terphényles, optionnellement substitués par deux groupes choisis indépendamment parmi les alkyles linéaires ou branchés en $C_1$ à $C_6$, les terphényles étant notamment des ortho-terphényles, méta-terphényles, ou para-terphényles, en particulier des para-terphényles ou des ortho-terphényles ;
- Les hydrocarbures aromatiques polycycliques choisis parmi naphtalènes, acénaphtènes, anthracènes, pyrènes, lesquels sont optionnellement substitués par deux groupes choisis indépendamment parmi les alkyles linéaires ou branchés en $C_1$ à $C_6$ ;
- Les fluorènes et carbazoles, lesquels sont optionnellement substitués par deux ou quatre groupes choisis indépendamment parmi les alkyles linéaires ou branchés en $C_1$ à $C_6$ ;
- Les 2,5-diphenyloxazoles, optionnellement substitués par deux groupes choisis indépendamment parmi les alkyles linéaires ou branchés en $C_1$ à $C_6$ ;
- Les composés de formule (I) :

[Chem. 1]

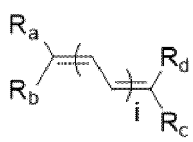

,

dans laquelle :

i est égal à 0 ou 1 ;
$R_a$, $R_b$, $R_c$ et $R_d$ sont indépendamment choisis parmi H et phényle, au moins deux des groupes $R_a$, $R_b$, $R_c$ et $R_d$ étant un phényle ;
lesdits composés de formule (I) étant optionnellement substitués, en particulier sur les groupes phényle, par deux groupes choisis indépendamment parmi les alkyles linéaires ou branchés en $C_1$ à $C_6$ ;

- Le triphénylméthane, optionnellement substitué, en particulier sur chaque groupe phényle, par un groupe choisi indépendamment parmi les alkyles linéaires ou branchés en $C_1$ à $C_6$ ;
- La tribenzylamine, optionnellement substituée, en particulier sur chaque groupe phényle, par un groupe choisi indépendamment parmi les alkyles linéaires ou branchés en $C_1$ à $C_6$ ;
- Le 2,5-dibenzylphénol, optionnellement substitué, en particulier sur chaque atome de carbone en alpha des groupes phényle, par un ou deux groupes choisis indépendamment parmi les alkyles linéaires ou branchés en $C_1$ à $C_6$, le 2,5-dibenzylphénol substitué étant en particulier le 2,5-bis(2-phénylpropan-2-yl)phénol ;

ladite composition n'étant pas constituée de biphényle et naphtalène.

L'invention concerne en particulier une composition constituée de ou comprenant au moins deux composés choisis parmi :

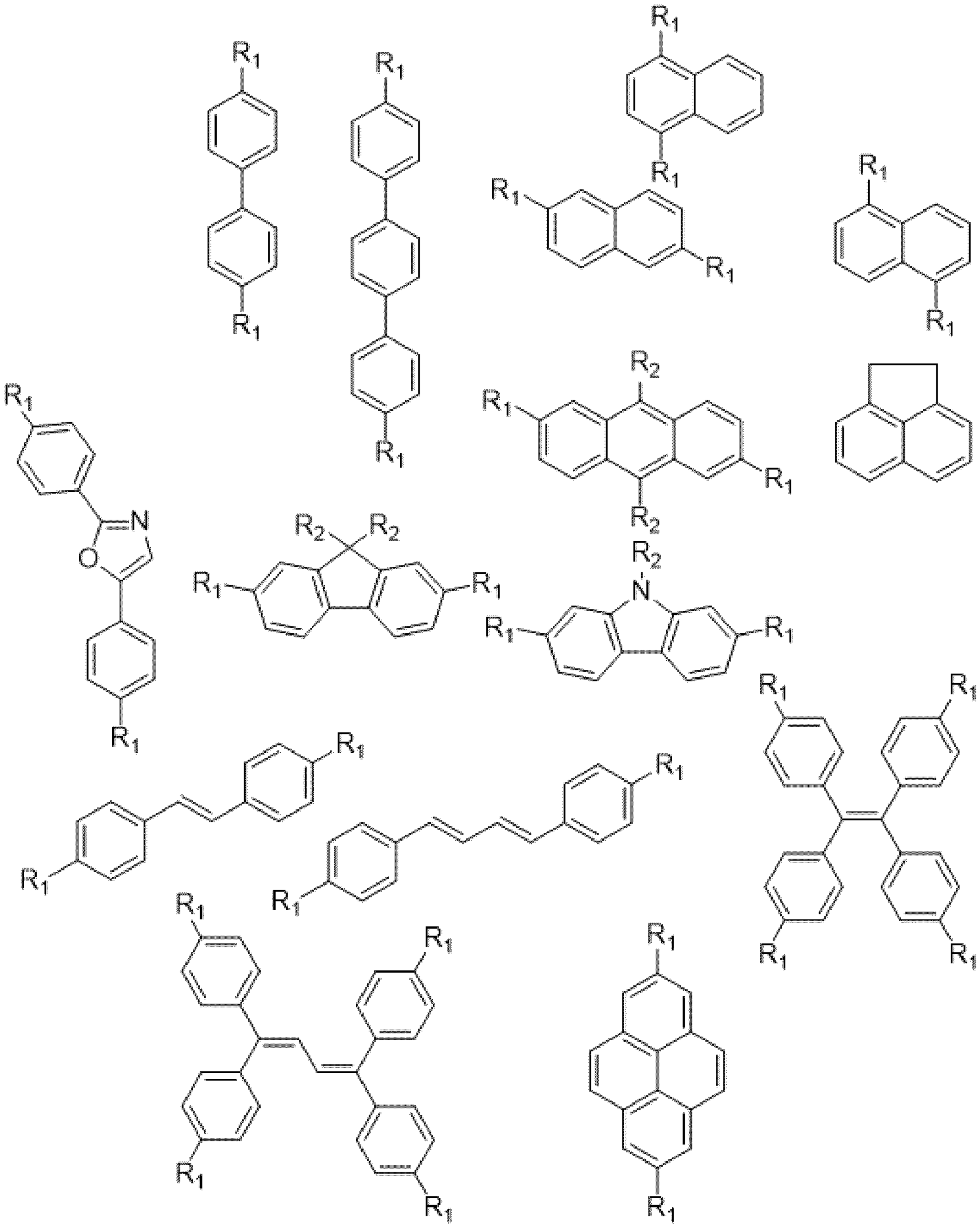

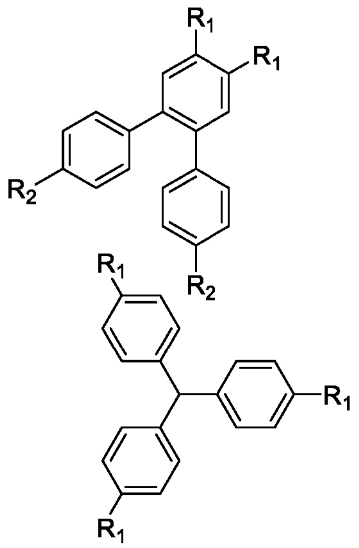

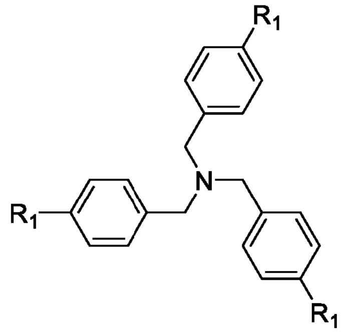

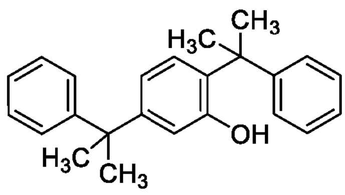

dans lesquelles $R_1$ et $R_2$ sont choisis indépendamment d'un composé à l'autre,
$R_1$ étant choisi parmi H et les alkyles linéaires ou branchés en $C_1$ à $C_6$, et $R_2$ étant choisi parmi H et les alkyles linéaires ou branchés en $C_1$ à $C_6$.

Le di-tert-butyl-benzène est notamment le 1,4-di-tert-butyl-benzène.

Les biphényle et terphényles sont notamment le biphényle, le *para*-terphényle ou le *méta*-terphényle. Les biphényles optionnellement substitués sont notamment de formule suivante :

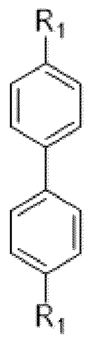

,

dans laquelle $R_1$ est choisi parmi H et les alkyles linéaires ou branchés en $C_1$ à $C_6$.

Les terphényles optionnellement substitués sont notamment de formule suivante :

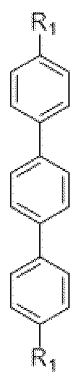

,

dans laquelle $R_1$ est choisi parmi H et les alkyles linéaires ou branchés en $C_1$ à $C_6$, ou de formule suivante :

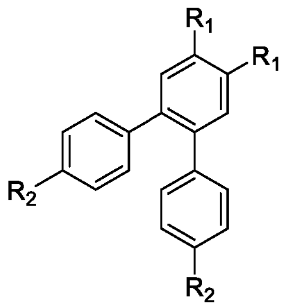

,

dans lesquelles $R_1$ et $R_2$ sont choisis indépendamment d'un composé à l'autre, $R_1$ étant choisi parmi H et les alkyles linéaires ou branchés en $C_1$ à $C_6$, et $R_2$ étant choisi parmi H et les alkyles linéaires ou branchés en $C_1$ à $C_6$.

Les naphtalènes optionnellement sont notamment d'une des formules suivantes :

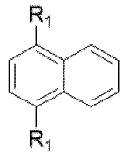

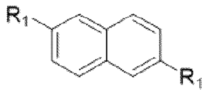

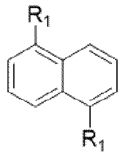

dans laquelle $R_1$ est choisi parmi H et les alkyles linéaires ou branchés en $C_1$ à $C_6$.

L'acénaphtène est notamment non substitué.

Les anthracènes optionnellement substitués sont notamment de formule suivante :

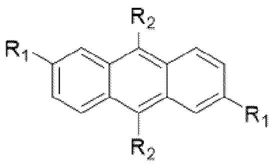

,

dans laquelle $R_1$ est choisi parmi H et les alkyles linéaires ou branchés en $C_1$ à $C_6$, et $R_2$ est choisi parmi H et les alkyles linéaires ou branchés en $C_1$ à $C_6$.

Les pyrènes optionnellement sont notamment de formule suivante :

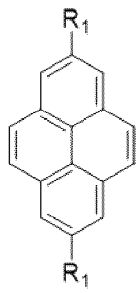

,

dans laquelle $R_1$ est choisi parmi H et les alkyles linéaires ou branchés en $C_1$ à $C_6$.

Les 2,5-diphenyloxazoles optionnellement sont notamment de formule suivante :

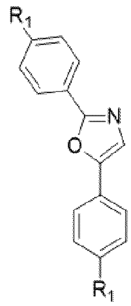

,

dans laquelle $R_1$ est choisi parmi H et les alkyles linéaires ou branchés en $C_1$ à $C_6$.

Les fluorènes optionnellement substitués sont notamment de formule suivante :

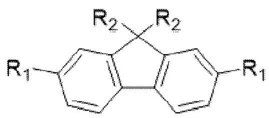

,

dans laquelle $R_1$ est choisi parmi H et les alkyles linéaires ou branchés en $C_1$ à $C_6$, et $R_2$ est choisi parmi H et les alkyles linéaires ou branchés en $C_1$ à $C_6$.

Les carbazoles optionnellement substitués sont notamment de formule suivante :

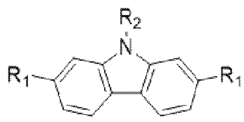

,

dans laquelle $R_1$ est choisi parmi H et les alkyles linéaires ou branchés en $C_1$ à $C_6$, et $R_2$ est choisi parmi H et les alkyles linéaires ou branchés en $C_1$ à $C_6$.

Les composés de formule (I) sont notamment choisis parmi les 1,2-diphényléthènes, 1,1,2,2-tetraphényléthènes, 1,2-diphénylbutadiènes, 1, 1,4,4-tetraphénylbutadiènes, optionnellement substitués, en particulier sur les groupes phényle, par deux groupes choisis indépendamment parmi les alkyles linéaires ou branchés en $C_1$ à $C_6$.

Les 1,2-diphényléthènes optionnellement substitués sont notamment de formule suivante :

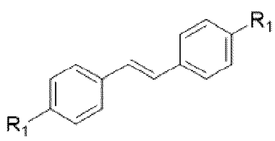

,

dans laquelle $R_1$ est choisi parmi H et les alkyles linéaires ou branchés en $C_1$ à $C_6$.

Les 1,1,2,2-tetraphényléthènes optionnellement substitués sont notamment de formule suivante :

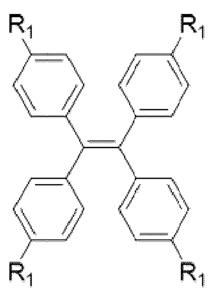

,

dans laquelle $R_1$ est choisi parmi H et les alkyles linéaires ou branchés en $C_1$ à $C_6$.

Les 1,2-diphénylbutadiènes optionnellement substitués sont notamment de formule suivante :

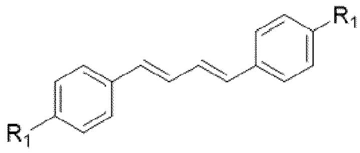

,

dans laquelle $R_1$ est choisi parmi H et les alkyles linéaires ou branchés en $C_1$ à $C_6$.
Les 1,1,4,4-tetraphénylbutadiènes optionnellement substitués sont notamment de formule suivante :

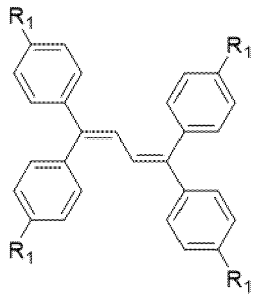

,

dans laquelle $R_1$ est choisi parmi H et les alkyles linéaires ou branchés en $C_1$ à $C_6$.
Le triphénylméthane, optionnellement substitué, est notamment de formule suivante :

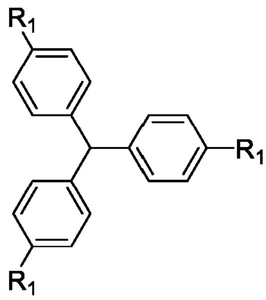

,

dans laquelle $R_1$ est choisi parmi H et les alkyles linéaires ou branchés en $C_1$ à $C_6$.
La tribenzylamine, optionnellement substituée, est notamment de formule suivante :

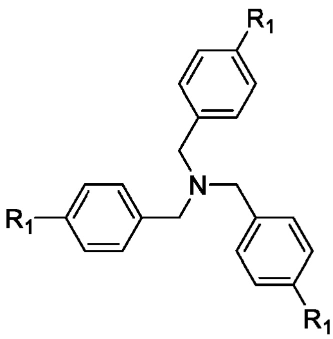

,

dans laquelle $R_1$ est choisi parmi H et les alkyles linéaires ou branchés en $C_1$ à $C_6$.
Le 2,5-dibenzylphénol est en particulier le 2,5-bis(2-phénylpropan-2-yl)phénol de formule suivante :

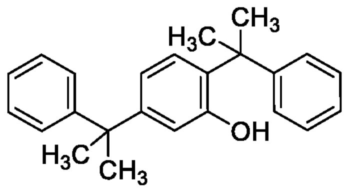

.

La composition selon l'invention peut être une composition dans laquelle les aux moins deux composés sont d'une même formule telle que définie précédemment, différant uniquement de par la signification du groupe $R_1$ et/ou $R_2$.

Selon un mode de réalisation particulier, la composition selon l'invention est constituée d'au moins deux composés tels que définis précédemment.

La composition, en particulier l'eutectique, selon l'invention, peut être binaire (à deux composants), tertiaire (à trois composants), quaternaire (à quatre composants), quinquénaire (à cinq composants, et ainsi de suite.

Selon un mode de réalisation, la composition de l'invention est tertiaire, quaternaire, ou quinquénaire. Ceci est susceptible de permettre l'obtention, si désiré, de compostions à la température de fusion plus basse, par exemple bien plus basse que la température ambiante.

Selon un mode de réalisation particulier, la composition est constituée de ou comprend :

- un biphényle, optionnellement substitué par deux groupes choisis indépendamment parmi les alkyles linéaires ou branchés en $C_1$ à $C_6$, en particulier le biphényle ; et
- un 2,5-diphenyloxazole, optionnellement substitué par deux groupes choisis indépendamment parmi les alkyles linéaires ou branchés en $C_1$ à $C_6$, en particulier le 2,5-diphenyloxazole.

Selon un mode de réalisation particulier, la composition est constituée de ou comprend :

- un biphényle, optionnellement substitué par deux groupes choisis indépendamment parmi les alkyles linéaires ou branchés en $C_1$ à $C_6$, en particulier le biphényle ;
- un 2,5-diphenyloxazole, optionnellement substitué par deux groupes choisis indépendamment parmi les alkyles linéaires ou branchés en $C_1$ à $C_6$, en particulier le 2,5-diphenyloxazole ; et
- un carbazole, optionnellement substitué par deux groupes choisis indépendamment parmi les alkyles linéaires ou branchés en $C_1$ à $C_6$, en particulier le 9-éthylcarbazole.

Selon un mode de réalisation particulier, la composition est constituée de ou comprend :

- un biphényle, optionnellement substitué par deux groupes choisis indépendamment parmi les alkyles linéaires ou branchés en $C_1$ à $C_6$, en particulier le biphényle ;
- un 2,5-diphenyloxazole, optionnellement substitué par deux groupes choisis indépendamment parmi les alkyles linéaires ou branchés en $C_1$ à $C_6$, en particulier le 2,5-diphenyloxazole ;
- un carbazole, optionnellement substitué par deux groupes choisis indépendamment parmi les alkyles linéaires ou branchés en $C_1$ à $C_6$, en particulier le 9-éthylcarbazole ; et
- un diterbutylbenzène, en particulier le 1,4- Diterbutylbenzène, un deuxième biphényle, optionnellement substitué par deux groupes choisis indépendamment parmi les alkyles linéaires ou branchés en $C_1$ à $C_6$, différent du premier, en particulier le diterbutylbiphényle, et/ou un fluorène, en particulier le diterbutylfluorène.
- Selon un mode de réalisation particulier, la composition est constituée de ou comprend :triphénylméthane,
- o-terphényle,
- tribenzylamine, et
- 2,4-bis(alpha,alpha-diméthylbenyl)phénol.

Selon un autre aspect, l'invention concerne également, l'utilisation d'une composition telle que décrite précédemment en tant que solvant.

Tous les modes de réalisation définis précédemment à propos de la composition de l'invention s'appliquent également ici, seuls ou en combinaisons.

Selon un mode de réalisation particulier, ladite composition n'étant pas constituée de biphényle et naphtalène. En effet, leur eutectique binaire peut ne pas avoir un point de fusion assez bas suivant l'utilisation visée.

Ces compositions, en tant que solvants, peuvent être utilisés dans toute les techniques d'extraction et/ou de séparation bien connues de l'homme du métier faisant intervenir un solvant organique.

**EXEMPLES**

**Exemple 1 : Préparation de compositions selon l'invention**

Les mélanges binaire $B_A$, ternaire $T_A$, et quaternaires $Q_A$ $Q_B$ et $Q_C$ ont été préparés et caractérisés comme suit.
La température de fusion des mélanges a été déterminée par Calorimétrie Différentielle à Balayage (DSC).

| Molécule | Proportion molaire | $T_{fusion}$ (°C) |
|---|---|---|
| Biphenyle | 44 % | 69 |
| Diphenyleoxazole (PPO) | 56 % | 70 |
| Eutectique | | $T_{eutectique}$ (°C) |
| $B_A$ | | 39 |

| Molécule | Proportion molaire | $T_{fusion}$ |
|---|---|---|
| Biphenyle | 35.0 % | 69 |
| 9-ethylcarbazole | 37,5 % | 70 |
| Diphenyleoxazole (PPO) | 27.5 % | 70 |
| Eutectique | | $T_{eutectique}$ (°C) |
| $T_A$ | | 19 |

| Molécule | Proportion molaire | $T_{fusion}$ |
|---|---|---|
| Biphenyle | 31,6 % | 69 |
| 9-ethylcarbazole | 33,7 % | 70 |
| Diphenyleoxazole (PPO) | 24,8% | 70 |
| Diterbutylbenzene (DTBB) | 9,9 % | 77 |
| Eutectique | | $T_{eutectique}$ (°C) |
| $Q_A$ | | 16 |

| Molécule | Proportion molaire | $T_{fusion}$ |
|---|---|---|
| Biphenyle | 32,5 % | 69 |
| 9-ethylcarbazole | 34,7 % | 70 |
| Diphenyleoxazole (PPO) | 25,6% | 70 |
| Diterbutybiphenyle (DTBP) | 7,3 % | 127 |
| Eutectique | | $T_{eutectique}$ (°C) |
| $Q_B$ | | 16 |

| Molécule | Proportion molaire | $T_{fusion}$ |
|---|---|---|
| Biphenyle | 32,6 % | 69 |
| 9-ethylcarbazole | 34,8 % | 70 |
| Diphenyleoxazole (PPO) | 25,6 % | 70 |
| Diterbutylfluorene (DTBF) | 7,0 % | 123 |
| Eutectique | | $T_{eutectique}$ (°C) |
| $Q_C$ | | 15 |

**Exemple** 2 : **Utilisation des compositions selon l'invention en tant que solvant pour la séparation liquide-liquide**

Le mélange $Q_A$ de l'exemple 1 a été évalué dans l'extraction d'un colorant en phase aqueuse. Le colorant est par exemple la coumarine 151.
Pour ce faire, de la coumarine 151 a été dissoute dans de l'eau, à pH 10. La solution ainsi obtenue est fluorescente (dans le vert).
Lors de l'extraction de cette phase aqueuse avec une quantité égale de toluène, seule une extraction partielle du colorant par le toluène a été visualisée (fluorescence verte restante dans la phase aqueuse).
En revanche, lors de l'extraction de cette même phase aqueuse avec le mélange $Q_A$, aucune fluorescence verte résiduelle n'a été observée au niveau de la phase aqueuse extraite, ce qui signifie une extraction totale de ce colorant du

milieu aqueux.

**Exemple 3 : Préparation de compositions selon l'invention et évaluation de l'extraction à l'aide desdites compositions**

Des mélanges quaternaires comprenant les composés suivants ont été préparés et caractérisés :

- triphénylméthane,
- o-terphényle,
- n,n-dibenzylaniline, et
- 2,4-bis(alpha,alpha-diméthylbenyl)phénol,

Et en particulier :

- 16,3 mol% triphénylméthane,
- 40,6 mol% o-terphényle,
- 21,1 mol% tribenzylamine,
- 22,8 mol% 2,4-bis(alpha,alpha-diméthylbenyl)phénol.

La température de fusion de ces composés a été déterminée par DSC, et la température de fusion du mélange calculé à l'aide de l'équation de Schrödler-van Larr :

- triphenylmethane : 93,6°C et 19,6 kJ/mol,
- o-terphényle : 56,9°C et 16,1 kJ/mol,
- tribenzylamine: 69,3°C et 22,4 kJ/mol,
- 2,4-bis(alpha,alpha-dimethylbenyl)phénol: 64,9°C et 22,9 kJ/mol.

Ceci donne une température de fusion pour l'eutectique de 13°C.

Des tests d'extractions à l'aide de cette composition ont été réalisé sur 5 composés. L'évaluation de l'extraction a été réalisé par spectroscopie UV-visible.

Le coefficient de partage de la molécule à extraire entre les phase organique (solvant eutectique hydrophobe) et aqueuse $K_{org/aq}$ a été calculé à l'aide de l'équation suivante :

$$K_{org/aq} = ( ([C_{aq,i}]-[C_{aq,\,f}]) * Vol_{aq}) / ( [C_{aq,f}] * Vol_{org}),$$

où :

$[C_{aq,i}]$ et $[C_{aq,f}]$ sont les concentrations avant et après extraction dans la phase aqueuse, et
$Vol_{aq}$ et $Vol_{org}$, sont les volumes des phases aqueuse et organique.

Les molécules à extraire et le coefficient de partage correspondant ainsi calculé sont indiqués ci-dessous :

- Bisphénol-A (polluant poly-aromatique utilisé comme additif dans les polymères, interdit pour certaines applications à cause de sa toxicité): K = 10 ;
- Nicotine (molécule cancérigène aromatique): K = 45 ;
- Lidocaine (médicament, aromatique) : K > 40 ;
- 2-naphthol (polyaromatique) : K = 50 ;
- Rhodamine B (colorant, polyaromatique) : K = 65.

## Revendications

**1.** Composition constituée de ou comprenant au moins deux composés choisis parmi :

- Les di-tert-butyl-benzènes;
- Les biphényles et terphényles, optionnellement substitués par deux groupes choisis indépendamment parmi les alkyles linéaires ou branchés en $C_1$ à $C_6$, les terphényles étant notamment des ortho-terphényles, méta-terphényles, ou para-terphényles, en particulier des para-terphényles ou des ortho-terphényles ;
- Les hydrocarbures aromatiques polycycliques choisis parmi naphtalènes, acénaphtènes, anthracènes, pyrènes, lesquels sont optionnellement substitués par deux groupes choisis indépendamment parmi les alkyles linéaires ou branchés en $C_1$ à $C_6$ ;
- Les fluorènes et carbazoles, lesquels sont optionnellement substitués par deux ou quatre groupes choisis indépendamment parmi les alkyles linéaires ou branchés en $C_1$ à $C_6$ ;
- Les 2,5-diphenyloxazoles, optionnellement substitués par deux groupes choisis indépendamment parmi les alkyles linéaires ou branchés en $C_1$ à $C_6$ ;
- Les composés de formule (I) :

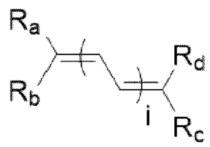
,

dans laquelle :

i est égal à 0 ou 1 ;
$R_a$, $R_b$, $R_c$ et $R_d$ sont indépendamment choisis parmi H et phényle, au moins deux des groupes $R_a$, $R_b$, $R_c$ et $R_d$ étant un phényle ;
lesdits composés de formule (I) étant optionnellement substitués, en particulier sur les groupes phényle, par deux groupes choisis indépendamment parmi les alkyles linéaires ou branchés en $C_1$ à $C_6$ ;

- Le triphénylméthane, optionnellement substitué, en particulier sur chaque groupe phényle, par un groupe choisi indépendamment parmi les alkyles linéaires ou branchés en $C_1$ à $C_6$ ;
- La tribenzylamine, optionnellement substituée, en particulier sur chaque groupe phényle, par un groupe choisi indépendamment parmi les alkyles linéaires ou branchés en $C_1$ à $C_6$ ;
- Le 2,5-dibenzylphénol, optionnellement substitué, en particulier sur chaque atome de carbone en alpha des groupes phényle, par un ou deux groupes choisis indépendamment parmi les alkyles linéaires ou branchés en $C_1$ à $C_6$, le 2,5-dibenzylphénol substitué étant en particulier le 2,5-bis(2-phénylpropan-2-yl)phénol ;

ladite composition n'étant pas constituée de biphényle et naphtalène.

**2.** Composition selon la revendication 1, laquelle est eutectique.

**3.** Composition selon la revendication 1 ou 2, dans laquelle les au moins deux composés sont notamment choisis parmi les composés suivants :

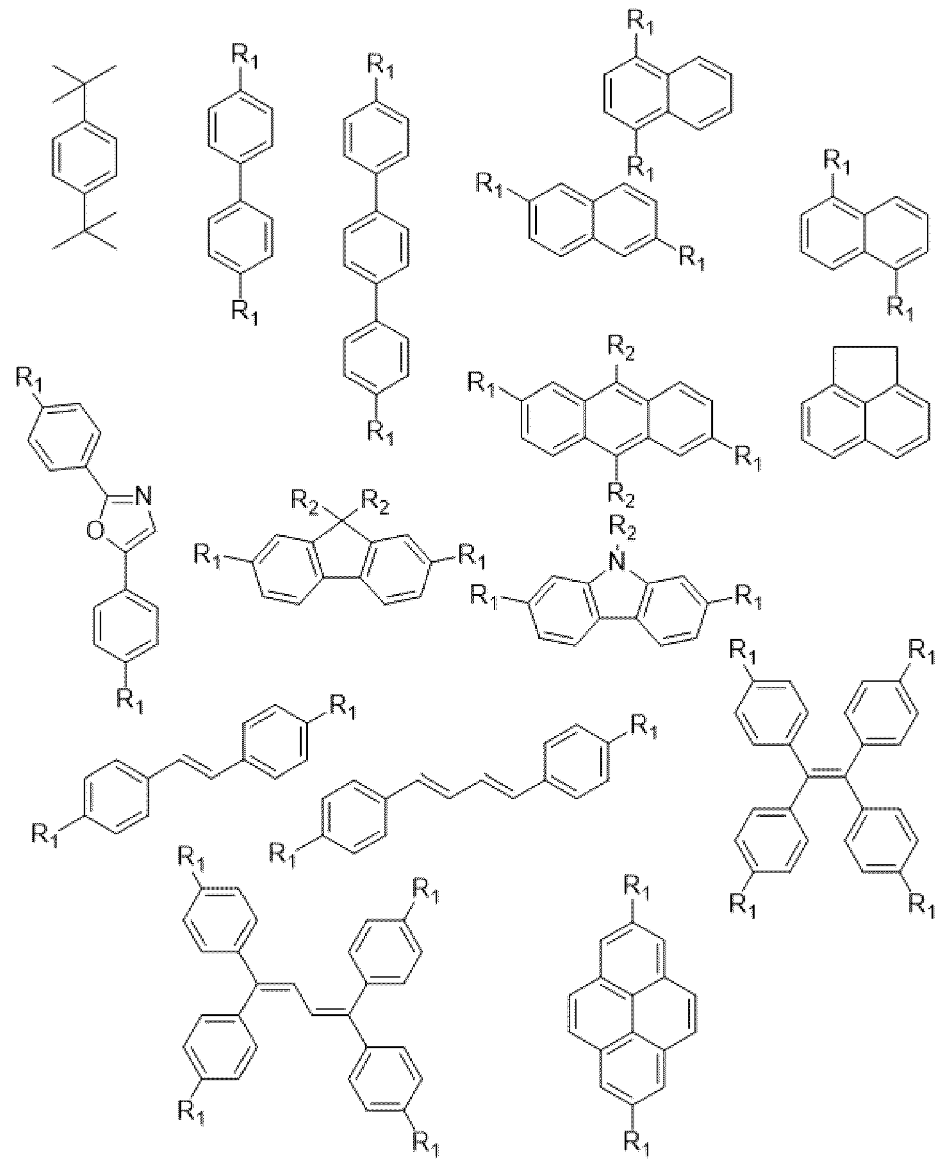

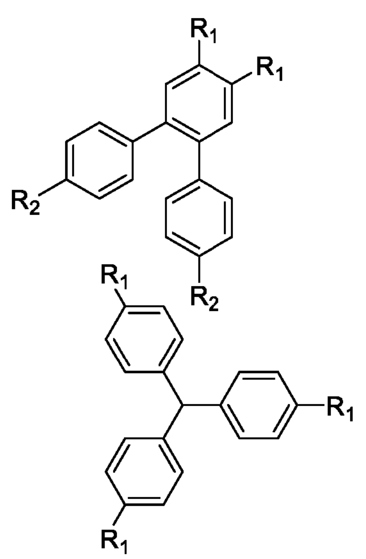

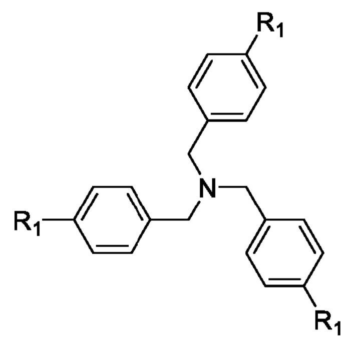

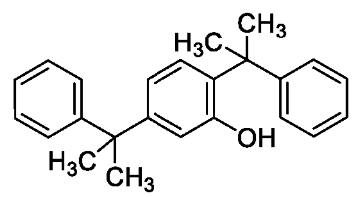

dans lesquelles $R_1$ et $R_2$ sont choisis indépendamment d'un composé à l'autre,
$R_1$ étant choisi parmi H et les alkyles linéaires ou branchés en $C_1$ à $C_6$, et $R_2$ étant choisi parmi H et les alkyles linéaires ou branchés en $C_1$ à $C_6$.

**4.** Composition selon l'une quelconque des revendications précédentes, laquelle est binaire, tertiaire, quaternaire, ou quinquénaire.

**5.** Composition selon l'une quelconque des revendications précédentes, laquelle est :

- binaire, et les deux composés de ladite composition sont présents dans celle-ci à hauteur de 20 à 80 %molaire, en particulier à hauteur de 40 à 60 %molaire ;
- tertiaire, et les trois composés de ladite composition sont présents dans celle-ci à hauteur de 10 à 55 %molaire, en particulier à hauteur de 25 à 40 %molaire ;
- quaternaire, et les quatre composés de ladite composition sont présents dans celle-ci à hauteur de 5 à 45 % molaire, en particulier à hauteur de 22 à 40 %molaire pour trois d'entre eux, et à hauteur de 6 à 34 %molaire pour le dernier ; ou
- quinquénaire, et les cinq composés de ladite composition sont présents dans celle-ci à hauteur de 5 à 45 % molaire.

**6.** Utilisation d'une composition en tant que solvant, ladite composition étant constituée de ou comprenant au moins deux composés choisis parmi :

- Les di-tert-butyl-benzènes;
- Les biphényles et terphényles, optionnellement substitués par deux groupes choisis indépendamment parmi les alkyles linéaires ou branchés en $C_1$ à $C_6$, les terphényles étant notamment des ortho-terphényles, méta-terphényles, ou para-terphényles, en particulier des para-terphényles ou des ortho-terphényles ;
- Les hydrocarbures aromatiques polycycliques choisis parmi naphtalènes, acénaphtènes, anthracènes, pyrènes, lesquels sont optionnellement substitués par deux groupes choisis indépendamment parmi les alkyles linéaires ou branchés en $C_1$ à $C_6$ ;
- Les fluorènes et carbazoles, lesquels sont optionnellement substitués par au moins deux groupes choisis indépendamment parmi les alkyles linéaires ou branchés en $C_1$ à $C_6$ ;
- Les 2,5-diphenyloxazoles, optionnellement substitués par deux groupes choisis indépendamment parmi les alkyles linéaires ou branchés en $C_1$ à $C_6$ ;
- Les composés de formule (I) :

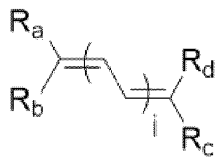
,

dans laquelle :

i est égal à 0 ou 1 ;
$R_a$, $R_b$, $R_c$ et $R_d$ sont indépendamment choisis parmi H et phényle, au moins deux des groupes $R_a$, $R_b$, $R_c$ et $R_d$ étant un phényle ;
lesdits composés de formule (I) étant optionnellement substitués, en particulier sur les groupes phényle, par deux groupes choisis indépendamment parmi les alkyles linéaires ou branchés en $C_1$ à $C_6$ ;

- Le triphénylméthane, optionnellement substitué, en particulier sur chaque groupe phényle, par un groupe choisi indépendamment parmi les alkyles linéaires ou branchés en $C_1$ à $C_6$ ;
- La tribenzylamine, optionnellement substituée, en particulier sur chaque groupe phényle, par un groupe choisi indépendamment parmi les alkyles linéaires ou branchés en $C_1$ à $C_6$ ;
- Le 2,5-dibenzylphénol, optionnellement substitué, en particulier sur chaque atome de carbone en alpha des groupes phényle, par un ou deux groupes choisis indépendamment parmi les alkyles linéaires ou branchés en $C_1$ à $C_6$, le 2,5-dibenzylphénol substitué étant en particulier le 2,5-bis(2-phénylpropan-2-yl)phénol.

**7.** Utilisation selon la revendication 6, dans laquelle ladite composition est dénuée de d'un autre solvant, l'autre solvant étant différent de tous les composés définis à la revendication 1 et de leurs mélanges.

**8.** Utilisation selon l'une quelconque des revendications 6 à 7, pour la séparation liquide-liquide, la séparation liquide-liquide-liquide, la séparation solide liquide, ou la séparation solide-liquide-liquide.

**9.** Utilisation selon l'une quelconque des revendications 6 à 9, pour la séparation liquide-liquide, l'une des phases liquides étant une phase aqueuse, l'autre phase liquide comprenant ou étant constituée de ladite composition.

**10.** Utilisation selon l'une quelconque des revendications 6 à 9, pour l'extraction d'un composé, ledit composé étant en particulier choisi parmi les composés organiques et les métaux, les composés organiques étant notamment des composés aromatiques ou hétéroaromatiques, ou comprenant des groupes aromatiques ou hétéroaromatiques, lesdits composés organiques, notamment aromatiques ou hétéroaromatiques, ou comprenant des groupes aromatiques ou hétéroaromatiques sont par exemple choisis parmi :

- Les composés phytosanitaires,
- Les polluants organiques, en particulier persistants, par exemple les dioxines,
- Les microplastiques,
- Les médicaments,
- Les colorants, et
- Le noir de carbone.